# EUROPEAN PATENT APPLICATION

(11) **EP 4 706 667 A1**
(43) Date of publication of application: **11.03.2026**
(21) Application number: 24198778.3
(22) Date of filing: 05.09.2024
(51) Int. Cl.: A61K 35/15, A61K 35/17, A61K 47/00, A61P 17/02, C12N 5/00

(54) **PHARMACEUTICAL COMPOSITION FOR TREATING WOUNDS**

(71) Applicant: Aposcience AG, 1200 Wien (AT)
(72) Inventor: Ankersmit, Jan Hendrik, 1060 Wien (AT); Mildner, Michael, 1060 Wien (AT)
(74) Representative: Schwarz & Partner Patentanwälte GmbH

(57) **Abstract**

The present invention relates to a pharmaceutical composition comprising a supernatant of a peripheral blood mononuclear cell (PBMC) culture for use in the treatment of a wound and/or for promoting wound healing or wound closure, wherein the supernatant is obtainable by cultivating PBMCs in a culture medium for at least 4 h, wherein the PBMC cell culture is subjected to ionizing radiation at a dose of at least 10 Gy before or during cultivation, wherein the composition is applied topically on the wound at a dose equivalent to a supernatant of 20×10⁶ to 50×10⁶ PBMCs/ml culture medium per cm² wound area.

## Description

### TECHNICAL FIELD

The present invention relates to the field of wound treatment.

### BACKGROUND ART

Wound healing is a complex physiological process that involves the coordinated interplay of various biological mechanisms, including hemostasis, inflammation, proliferation, and remodeling. Significant advances in wound care management have been made over the past few decades, particularly due to the development of advanced dressings, biologics, and other therapeutic interventions. However, despite these improvements, there remains a substantial unmet need for effective methods and therapeutic approaches in wound healing, especially in the context of chronic wounds such as diabetic ulcers and pressure sores. Chronic wounds affect millions of individuals worldwide and lead to significant healthcare costs and diminished quality of life.

Transplantation of adult stem cells has been proposed and tested to repair different kinds of tissue damage, since stem cells are known to transdifferentiate into various cells types, including skin cells. Despite very promising pre-clinical results, meticulously performed first clinical trials did not fully meet the expectations and the use of adult stem cell therapy for organ regeneration has not entered the routine clinical practice.

In a more recent approach, Timmers et al. (Stem Cell Res 1(2007): 129-137) demonstrated that fractionated conditioned medium products of mesenchymal stem cells and containing only soluble factors smaller that 100-200nm in size provide cardio protection in an ischemia reperfusion (I/R) injury model. Thus, it was thought that such preparations may contribute to wound healing processes.

Hacker S et al. (Sci Rep 6(2016): 25168) found that paracrine factors present in the supernatant of irradiated peripheral blood mononuclear cells (PBMCs) improve skin regeneration and angiogenesis in a porcine burn model demonstrating that such supernatants may be used in the treatment of wounds.

Clinical studies have shown that no wound closure progression in wounds treated with supernatants of irradiated PBMCs compared to wounds treated with placebo could be achieved under the conditions chosen (Simader E et al. Sci Rep. (2017) 7: 6216).

Hence, it is an object of the present invention to provide conditions under which supernatants of irradiated PBMCs result in a wound closure and, thus, in an effective treatment of wounds.

### SUMMARY OF THE INVENTION

Thus, the present invention relates to a pharmaceutical composition comprising a supernatant of a peripheral blood mononuclear cell (PBMC) culture for use in the treatment of a wound and/or for promoting wound healing or wound closure, wherein the supernatant is obtainable by cultivating PBMCs in a culture medium for at least 4 h, wherein the PBMC cell culture is subjected to ionizing radiation at a dose of at least 10 Gy before or during cultivation, wherein the composition is applied topically on the wound at a dose equivalent to a supernatant of 20×10⁶ to 50×10⁶ PBMCs/ml culture medium per cm² wound area.

It turned surprisingly out that the application of a certain dose of a supernatant of a PBMC culture on a wound promotes wound healing and wound closure and, thus, results in the treatment of wounds. The application of a composition comprising the supernatant of the present invention leads to a wound closure which cannot be achieved using standard of care products or other compositions known to promote the healing process of wounds. It turned out that the composition of the present invention enhances significantly the healing process if applied on a wound at a certain dose.

Another aspect of the present invention relates to a method for treating a wound and/or for promoting wound healing or wound closure comprising the steps of
a) providing a pharmaceutical composition comprising a supernatant of a peripheral blood mononuclear cell (PBMC) culture, wherein the supernatant is obtainable by cultivating PBMCs in a culture medium for at least 4 h, wherein the PBMC cell culture is subjected to ionizing radiation at a dose of at least 10 Gy before or during cultivation, and
b) applying topically the composition of a) on a wound at a dose equivalent to a supernatant of 20×10⁶ to 50×10⁶ PBMCs/ml culture medium per cm² wound area.

A further aspect of the present invention relates to a kit comprising
a) a container comprising a composition, preferably a lyophilized composition, comprising or consisting of a supernatant of a peripheral blood mononuclear cell (PBMC) culture, wherein the supernatant is obtainable by cultivating a PBMC cell culture for at least 4 h, wherein said PBMC cell culture is subjected to ionizing radiation at a dose of at least 10 Gy before or during cultivation, and
b) a container comprising an aqueous solution comprising 1 to 3 wt% of an alginate.

It is advantageous to store the composition, preferably the lyophilized composition, comprising or consisting of the supernatant of the PBMC culture separate from the aqueous solution comprising the alginate in order to increase the shelf life of the pharmaceutically active supernatant. Furthermore, the separate storage allows to adjust the concentration of the supernatant present in the pharmaceutical composition to be applied to a wound.

Another aspect of the present invention relates to a method for producing a pharmaceutical composition to treat a skin lesion by mixing
a) a composition comprising or consisting of a supernatant of a peripheral blood mononuclear cell (PBMC) culture, wherein the supernatant is obtainable by cultivating a PBMC cell culture for at least 4 h, wherein said PBMC cell culture is subjected to ionizing radiation at a dose of at least 10 Gy before or during cultivation, with
b) an aqueous solution comprising 1 to 3 wt% of an alginate
in a ratio from 1:1 to 1:5, preferably from 1:2 to 1:4, more preferably about 1:3.

### BRIEF DESCRIPTION OF THE FIGURES

Fig. 1 shows patients with ≥80% reduction in wound area from the start of the treatment.
Fig. 2 shows the percentage of complete wound closure of wounds of patients treated with various concentrations of APO-2 and placebo.

### DESCRIPTION OF EMBODIMENTS

The term "treat" or "treating" includes abrogating, substantially inhibiting, slowing or reversing the progression of a disease, condition, disorder or injury, substantially ameliorating clinical or esthetical symptoms of a disease, condition, disorder or injury, substantially preventing the appearance of clinical or esthetical symptoms of a disease, condition, disorder or injury, and protecting from harmful or annoying symptoms. The term "treat" or "treating", as used herein, further refers to accomplishing one or more of the following: (a) reducing the severity of the disease, condition, disorder or injury; (b) limiting development of symptoms characteristic of the disease, condition, disorder or injury being treated; (c) limiting worsening of symptoms characteristic of the disease, condition, disorder or injury being treated; (d) limiting recurrence of the disease, condition, disorder or injury in patients that have previously had the disease, condition, disorder or injury; and (e) limiting recurrence of symptoms in patients that were previously symptomatic for the disease, condition, disorder or injury.

The term "wound", as used herein, refers to a disruption of the normal continuity of structures caused by a physical (e.g. mechanical) force, a biological or a chemical means. The term "wound" includes, but is not limited to, incisional wounds, excisional wounds, traumatic wounds, lacerations, punctures, cuts and the like.

The term "wound healing", as used herein, refers to a regenerative process with the induction of a temporal and spatial healing program, including, but not limited to, the processes of inflammation, granulation, neovascularization, migration of fibroblast, endothelial and epithelial cells, extracellular matrix deposition, reepithelialization, and remodeling. "Promoting wound healing" means that these processes are started or enhanced by applying the composition of the present invention on the wound.

The term "wound closure", as used herein, refers to the healing of a wound such that the edges of the wound are rejoined to form a continuous barrier.

"A dose equivalent to a supernatant of 20×10⁶ to 50×10⁶ PBMCs/ml culture medium per cm² wound area", as used herein, means that the pharmaceutical composition of the present invention, that is applied to one cm² of a wound, comprises the supernatant which is obtainable by cultivating 20×10⁶ to 50×10⁶ PBMCs per ml culture medium under the conditions set forth herein. Thus, the pharmaceutical composition applied to one cm² of a wound comprises the components present in the supernatant obtainable by cultivating 20×10⁶ to 50×10⁶ PBMCs per ml culture medium under the conditions set forth herein. If the supernatant is obtained by cultivating, for instance, more than 50×10⁶ PBMCs per ml culture medium, the supernatant will be diluted accordingly or, in case the supernatant has been lyophilized, dissolved in an appropriate amount of an aqueous pharmaceutically acceptable composition.

The term "wound size" or "wound area", as used herein, refers to a physical measure of disruption of the normal continuity of structures caused by a physical (e.g., mechanical) force, a biological or a chemical means. The wound size and the wound area can be determined by methods known in the art. Particularly preferred to determine the wound size and the wound area is the use of a camera system, preferably a 3-dimensional camera system. Exemplary methods are described in Jorgensen LB et al. (Int Wound J. 2016 Aug; 13(4): 540-553). A particularly preferred method for determining the wound size/wound area is described in Gugerell A et al. (Trials. 2021; 22: 10).

PBMCs used to produce the supernatant of the present invention can be obtained from whole blood using methods known in the art, such as ficoll gradient, hypotonic lysis, etc. These methods are well known in the art (see e.g. WO 2010/079086).

The PBMCs present in the culture medium are subjected to ionizing radiation during or before cultivation or even before being added to the culture medium. The ionizing radiation can be gamma radiation (e.g. Caesium 137 gamma radiation) or photon radiation (e.g. from a light source, from a laser etc.), at a dose of at least 10 Gy.

The pharmaceutical composition of the present invention is preferably applied topically on the wound. However, the pharmaceutical composition can also be applied by injection whereby the injection occurs preferably into the tissue/skin surrounding the wound.

The wound to be treated can be a mammalian or human wound (e.g. a wound affecting the skin of a mammal or a human individual), preferably a human wound. It turned out that the pharmaceutical composition of the present invention can not only be used to treat a human wound or promote the healing of a human wound, but also wounds of mammals.

According to a preferred embodiment of the present invention the wound is a chronic wound or an acute wound.

The composition of the present invention can be used for treating chronic or acute wounds or for promoting their healing process and wound closure. In particular, chronic wounds need special attention since they are known to be treated usually over a long period of time.

According to a further preferred embodiment of the present invention the wound is a skin wound, preferably an ulcer, a burn, an abrasion, a laceration, a puncture wound, an incision or an avulsion.

The composition of the present invention can be used for treating any kind of wound irrespective of their cause. This is a surprising finding since it is known that wounds being the result of different causes may involve different biological processes. It is, however, most preferred to use the composition of the present invention in the treatment of ulcers.

"Ulcers", as used herein, refer to a lesion of the skin that is characterized by the formation of pus and necrosis (death of surrounding tissue) usually resulting from inflammation or ischemia.

Hence, according to a particularly preferred embodiment of the present invention the chronic wound is an ulcer, preferably a diabetic ulcer, more preferably a diabetic foot ulcer, or a pressure ulcer (decubitus).

Diabetic wounds and impaired wound healing represent clinical challenges with an unmet need. The process of wound healing involves well-orchestrated biological events, including macrophage invasion, reepithelization, fibroblast migration and proliferation, extracellular matrix deposition, and neo-angiogenesis. However, in diabetic wounds/ulcers, the healing process is impaired, which can lead to chronic non-healing wounds, such as diabetic foot ulcers, and, in the worst case, requires limb amputation. It was surprisingly found that the composition of the present invention applied on such wounds/ulcers at the defined dose leads to a complete wound closure.

It turned out that the composition can be used for the treatment of wounds of any size. However, the healing process is particularly fast, if the wound covers, according to a preferred embodiment of the present invention, an area of less than 10 cm², preferably of less than 8 cm², more preferably of less than 5 cm², more preferably of less than 4 cm².

According to another preferred embodiment of the present invention the wound covers an area of more than 0.1 cm², preferably of more than 0.2 cm², more preferably of more than 0.5 cm², more preferably of more than 0.8 cm², more preferably of more than 1 cm².

According to a further preferred embodiment of the present invention the wound covers an area from from 0.1 cm² to 10 cm², preferably from 0.1 cm² to 8 cm², preferably from 0.1 cm² to 5 cm², preferably from 0.1 cm² to 4 cm², preferably from 0.2 cm² to 4 cm², preferably from 0.2 cm² to 5 cm², preferably from 0.2 cm² to 8 cm², preferably from 0.2 cm² to 10 cm², preferably from 0.5 cm² to 4 cm², preferably from 0.5 cm² to 5 cm², 0.5 cm² to 8 cm², preferably from 0.5 cm² to 10 cm², preferably from 0.8 cm² to 4 cm², 0.8 cm² to 5 cm², preferably from 0.8 cm² to 8 cm², preferably from 0.8 cm² to 10 cm², preferably from 1 cm² to 4 cm², preferably from 1 cm² to 5 cm², preferably from 1 cm² to 8 cm², preferably from 1 cm² to 10 cm². According to a particularly preferred embodiment of the present invention the wound covers an area from 0.2 cm² to 5 cm², preferably from 0.2 cm² to 4 cm², preferably from 0.8 cm² to 4 cm², 0.8 cm² to 5 cm².

According to a preferred embodiment of the present invention the composition is applied to a wound for at least 8 days, preferably for at least 10 days, more preferably for at least 14 days, more preferably for at least 21 days, more preferably for at least 30 days.

The application of the composition of the present invention on wounds over several days is advantageous and results in a complete closure of the treated wound. Hence, it is preferred to apply the composition on a wound for at least 8 days.

According to a further preferred embodiment of the present invention the composition is applied to the wound twice a day, preferably once a day, more preferably every second day, more preferably every third day, more preferably every fourth day, more preferably every fifth day, more preferably every sixth day, more preferably every seventh day.

According to another preferred embodiment of the present invention the composition is applied to the wound for at least 8 days, twice a day; preferably for at least 8 days, once a day; preferably for at least 8 days, every second day; preferably for at least 8 days, every third day; preferably for at least 8 days, every fourth day; preferably for at least 8 days, every fifth day; preferably for at least 8 days, every sixth day; preferably for at least 8 days, every seventh day; preferably for at least 10 days, twice a day; preferably for at least 10 days, once a day; preferably for at least 10 days, every second day; preferably for at least 10 days, every third day; preferably for at least 10 days, every fourth day; preferably for at least 10 days, every fifth day; preferably for at least 10 days, every sixth day; preferably for at least 10 days, every seventh day; preferably for at least 14 days, twice a day; preferably for at least 14 days, once a day; preferably for at least 14 days, every second day; preferably for at least 14 days, every third day; preferably for at least 14 days, every fourth day; preferably for at least 14 days, every fifth day; preferably for at least 14 days, every sixth day; preferably for at least 14 days, every seventh day; preferably for at least 21 days, twice a day; preferably for at least 21 days, once a day; preferably for at least 21 days, every second day; preferably for at least 21 days, every third day; preferably for at least 21 days, every fourth day; preferably for at least 21 days, every fifth day; preferably for at least 21 days, every sixth day; preferably for at least 21 days, every seventh day; preferably for at least 30 days, twice a day; preferably for at least 30 days, once a day; preferably for at least 30 days, every second day; preferably for at least 30 days, every third day; preferably for at least 30 days, every fourth day; preferably for at least 30 days, every fifth day; preferably for at least 30 days, every sixth day; and preferably for at least 30 days, every seventh day.

According to a preferred embodiment of the present invention the composition is administered to the wound once a week, preferably twice a week, more preferably three times a week, more preferably four times a week, more preferably five times a week, more preferably six times a week, more preferably daily.

According to a particularly preferred embodiment of the present invention the composition is applied topically on the wound at a dose equivalent to a supernatant of 20×10⁶ to 40×10⁶ PBMCs/ml, preferably 20×10⁶ to 30×10⁶ PBMCs/ml, more preferably 22×10⁶ to 28×10⁶ PBMCs/ml, more preferably 24×10⁶ to 26×10⁶ PBMCs/ml, more preferably about 25×10⁶ PBMCs/ml, culture medium per cm² wound area.

According to a preferred embodiment of the present invention the composition comprises 0.5 to 5 wt%, preferably 1 to 4 wt% of the alginate, more preferably 2 to 3 wt% of the alginate, more preferably about 2.25 wt%, of an alginate.

The addition of alginate to the composition of the present invention is advantageous since the presence of alginate in the composition enhances the effect on the treatment of the wound to be treated and may additionally promote wound healing and, thus, wound closure. Furthermore, the presence of alginate in the composition of the present invention increases its viscosity which is advantageous when applying the composition on the wound to be treated.

According to another preferred embodiment of the present invention the alginate is a sodium alginate and/or a potassium alginate.

According to a further preferred embodiment of the present invention the alginate can be obtained from various sources. Alginate is a natural polysaccharide that is primarily obtained from the cell walls of brown seaweeds. Some of the main sources of alginate include species from the genus *Laminaria, Ascophyllum, Macrocystis* and *Sargassum.* Other sources of alginate can be other marine algae. Also certain microorganisms can be a source of alginate. Certain bacteria, for instance, can produce alginate as an exopolymer, whereby bacteria from the genus *Pseudomonas* and *Azotobacter* are known producers of alginate. However, the preferred alginate is of a marine source.

According to a preferred embodiment of the present invention the pharmaceutical composition comprises further 0.1 to 3 wt%, preferably 0.5 to 2 wt%, more preferably 0.5 to 1.5 wt%, more preferably 0.5 to 1 wt%, more preferably about 0.825 wt%, cellulose or at least one cellulose derivative.

Cellulose and cellulose derivatives in the composition of the present invention are helpful to adjust viscosity of the composition. A concentration of 0.1 to 3 wt% cellulose or at least one cellulose derivative in the composition of the present invention renders the composition sufficiently viscose to be applied topically on the wound.

According to another preferred embodiment of the present invention the at least on cellulose derivative is carboxymethyl cellulose (CMC) and/or hydroxyethyl cellulose.

According to a preferred embodiment of the present invention the pharmaceutical composition comprises further 10 to 25 wt%, preferably 15 to 20 wt%, more preferably about 18.25 wt%, of at least one diol, preferably propane-1,2-diol.

Diols and in particular propylene glycol serve in the composition of the present invention as moistening agent, as solvent for active components present in the supernatant and as a penetration enhancer for facilitating the delivery of active ingredients into the wound.

According to a further preferred embodiment of the present invention the PBMC cell culture comprises monocytes, T cells, B cells and/or NK cells.

According to a preferred embodiment of the present invention the PBMCs are cultivated in a culture mediuem, preferably in a cell culture medium selected from the group consisting of a cell growth medium, preferably CellGro medium, more preferably Cellgro GMP DC medium, RPMI, DMEM, X-vivo and Ultraculture.

The PBMCs may be cultivated in one of these culture media. It is also possible to use an alternative culture medium to obtain the supernatant of the present invention. Using alternative methods and culture media shall result in the production of a supernatant showing similar, identical or even better properties as supernatants obtained by cultivating PBMCs with cell culture media typically used for mammalian cell cultures (e.g. CellGro medium, Cellgro GMP DC medium, RPMI, DMEM, X-vivo and Ultraculture as used to obtain PBMC supernatants). The properties are measured using a potency assay as described in EP 3 729 079 B1, preferably potency assays involving the measurement of AP-1 promoter activities and/or the amount of phosphorylated HSP-27 as described therein.

A preferred method for cultivating PBMCs may comprise the step of incubating the PBMCs in a culture medium comprising at least one sodium salt, at least one potassium salt and at least calcium salt under an atmosphere comprising less than 1% carbon dioxide.

It turned out that the PBMCs can be cultivated under an atmosphere comprising less than 1% CO₂ using a cell culture medium comprising at least one sodium salt, at least one potassium salt and at least calcium salt. The use of such a culture medium allows to keep the pH in a range suitable for mammalian cells although the CO₂ concentration in the atmosphere, where the cells are cultivated, is low compared to typically used atmospheres/gases comprising 4 to 7% or approx. 5%, CO₂. This is particularly advantageous since a CO₂ incubator, which is typically used to cultivate mammalian cells, is not needed and even air (i.e. atmospheric air) can be used to cultivate mammalian cells without adding additional CO₂.

The atmosphere under which the PBMCs are cultivated my comprise less than 0.5%, preferably less than 0.2%, more preferably less than 0.1%, more preferably less than 0.05%, carbon dioxide. The atmosphere may also comprise oxygen and/or nitrogen, wherein said atmosphere comprises preferably 0.5 to 21%, more preferably 1 to 21%, even more preferably 5 to 21%, even more preferably 10 to 21%, oxygen and/or preferably 60 to 78%, more preferably 65 zo 78%, more preferably 70 to 78%, nitrogen. The atmosphere is particularly preferred air.

The culture medium preferably used to obtain the supernatant of the present invention may comprise 50 to 200 mmol/L, preferably 100 to 150 mmol/L, more preferably 125 to 135 mmol/L, more preferably 130 to 132 mmol/L, more preferably around 131 mmol/L, sodium ions.

"Around", as used herein, means that the concentration to which the term is associated to can be 10% higher or lower, preferably 5 % higher or lower, more preferably 4 % higher or lower, more preferably 3 % higher or lower, more preferably 2 % higher or lower, more preferably 1 % higher or lower.

The at least one sodium salt is preferably sodium chloride.

The culture medium may comprise 2 to 8 mmol/L, preferably 3 to 6 mmol/L, more preferably 4 to 6 mmol/L, more preferably 5 to 6 mmol/L, more preferably around 5.36 mmol/L, potassium ions, which are added to the culture medium as potassium chloride, for instance.

The culture medium may comprise 0.5 to 3 mmol/L, preferably 1 to 2 mmol/L, more preferably 1.5 to 2 mmol/L, more preferably around 1.84 mmol/L, calcium ions, which can be added as calcium chloride.

The culture medium to cultivate the PBMCs is preferably Ringer's solution (USP43-NF38 (2020), Monograph "Ringer's Injection", S. 3887).Ringer's solution may comprise further at least one salt of a C₂ to C₅ carbonic acid, wherein the C₂ to C₅ carbonic acid is preferably lactate, more preferably L-lactate, and may be added to the culture medium in a concentration of 10 to 50 mmol/L, preferably 15 to 40 mmol/L, more preferably 20 to 30 mmol/L, more preferably around 28 mmol/L. Hence, the culture medium is particularly preferred lactated Ringer's solution (USP43-NF38 (2020), Monograph "Lactated Ringer's Injection", S. 3891).

The Ringer's solution used as culture medium comprises preferably at least one polypeptide, preferably at least one blood polypeptide, most preferably albumin (e.g. serum albumin) or insulin, at a concentration of 1 mg/L to 50 g/L, preferably 2 mg/L to 40 g/L, more preferably 4 mg/L to 30 g/L, more preferably 1 g/L to 20 g/L, more preferably 2 g/L to 10 g/L.

According to a preferred embodiment of the present invention the PBMCs are incubated/cultivated in the culture medium at a temperature of 30°C to 38°C, preferably 35°C to 38 °C, in order to obtain the supernatant of the present invention.

According to a further preferred embodiment of the present invention the PBMCs are subjected to an ionizing radiation at a dose of at least 20 Gy, preferably at least 30 Gy, more preferably at least 40 Gy, more preferably at least 50 Gy.

According to another preferred embodiment of the present invention the PBMCs are cultivated for at least 6 h, preferably for at least 12 h, more preferably for at least 24 h, most preferably for around 24 h, before isolating its supernatant. The maximum cultivation time does preferably not exceed 72 h, preferably 60 h, more preferably 48 h, more preferably 36 h.

After the incubation of the stressed PBMCs under the conditions set forth herein the cells and cellular debris can be removed by centrifugation to obtain the supernatant and/or by filtrating the cultured cell culture through a filter (e.g. 0.2 µm filter).

Another aspect of the present invention relates to a method for treating a wound and/or for promoting wound healing or wound closure comprising the steps of
a) providing a pharmaceutical composition comprising a supernatant of a peripheral blood mononuclear cell (PBMC) culture, wherein the supernatant is obtainable by cultivating PBMCs in a culture medium for at least 4 h, wherein the PBMC cell culture is subjected to ionizing radiation at a dose of at least 10 Gy before or during cultivation, and
b) applying topically the composition of a) on a wound at a dose equivalent to a supernatant of 20×10⁶ to 50×10⁶ PBMCs/ml culture medium per cm² wound area.

Another aspect of the present invention relates to a kit comprising
a) container comprising a composition, preferably a lyophilized composition, comprising or consisting of a supernatant of a peripheral blood mononuclear cell (PBMC) culture, wherein the supernatant is obtainable by cultivating a PBMC cell culture for at least 4 h, wherein said PBMC cell culture is subjected to ionizing radiation at a dose of at least 10 Gy before or during cultivation, and
b) a container comprising an aqueous solution comprising 1 to 3 wt% of an alginate and optionally further components as described above.

A further aspect of the present invention relates to a method for producing a pharmaceutical composition to treat a wound and/or for promoting wound healing or wound closure by mixing
a) a composition comprising or consisting of a supernatant of a peripheral blood mononuclear cell (PBMC) culture, wherein the supernatant is obtainable by cultivating a PBMC cell culture for at least 4 h, wherein said PBMC cell culture is subjected to ionizing radiation at a dose of at least 10 Gy before or during cultivation, with
b) an aqueous solution comprising 1 to 3 wt% of an alginate and optionally further components as described above
in a ratio from 1:1 to 1:5, preferably from 1:2 to 1:4, more preferably about 1:3.

### EXAMPLE

### Materials & Methods

### Production of APO-2 and placebo

Blood obtained from the Austrian Red Cross Blood Transfusion Service of Upper Austria was used to produce APO-2. PBMCs were separated from whole blood samples by density centrifugation using LSM 1077 (Lymphocyte Separation Medium, Lonza, Switzerland). Removal of LSM was achieved by two washing steps using Dulbecco's phosphate-buffered saline (Lonza, Switzerland). PBMCs were resuspended in phenol red-free CellGro GMP DC medium (CellGenix, Freiburg, Germany) containing no xenogeneic proteins. A sample was drawn for complete blood count to adjust white blood cells to a concentration of 25×10⁶ cells/ml. Irradiation with 60Gy (gamma radiation) induced apoptosis of PBMCs. By cultivation of apoptotic PBMCs in CellGro GMP DC medium, release of the secretome was achieved. After incubation for 24h±2h, cells were removed by centrifugation. The supernatant containing the secretome was sterile filtered at a pore size of 0.22um. The adequate production of APO-2 was defined by appropriate secretion of the following important cytokines: interleukin (IL)-8 (0-5214pg/ml), epidermal growth factor (EGF; 25-226pg/ml), and transforming growth factor-β (TGF-β; 2575-21732pg/ml). The supernatant was finally lyophilised.

Lyophilized culture medium not containing any cells (CellGro, CellGenix, Freiburg, Germany) served as placebo.

Induction of apoptosis was determined before irradiation and after cultivation of the cells by fluorescence-activated cell sorting analysis using the FITC Annexin V Apoptosis Detection Kit (BD Biosciences, Franklin Lakes, NJ, US). Concentrations of IL-8/C×CL8 (C×C-motive-chemokine 8), EGF, and TGF-β were determined with enzyme-linked immunosorbent assay (ELISA) to verify successful production of APO-2 according to GMP definitions. Finally, the product underwent endotoxin, mycoplasma and sterility testing. Cell culture supernatant samples were additionally screened for herpes virus contamination via polymerase chain reaction.

### Production of the preparation to be applied on wounds

The preparation to be applied on the wound of the patients comprised 50 U supernatant/ml, 100 U supernatant/ml and 200 U supernatant/ml, respectively. 1 U corresponds to a supernatant obtained by cultivating 10⁶ PBMCs. The respective lyophilized product obtained as described above was dissolved in a 0.9% NaCl solution to the required concentration and combined in a ratio of 1:3 with a solution comprising 70 wt% water, 25 wt% propylene glycol, 3 wt% sodium alginate, 1 wt% carboxymethyl cellulose, 0.1 wt% hydroxyethyl cellulose and 0.1 wt% NaCl.

### Clinical Assessment

To assess the effectiveness of the APO-2 preparation on wound healing four groups of patients with diabetic foot ulcer present for ≥4 weeks, a foot ulcer Wagner Grade I-II or ARMSTRONG Grade I-A or II-A, and an estimated foot ulcer surface area ≥0.8 cm² and ≤8 cm², received three different doses of supernatant in a hydrogel matrix (12.5 U/ml/cm² wound area, 25 U/ml/cm² wound area and 50 U/ml/cm² wound area APO-2) and placebo (hydrogel matrix and fresh cell culture used to culture the PMBCs). At least 20% of the patients of each patient group had wounds with a wound area >4 cm².

In total 122 patients (27 [12.5 U/mL], 38 [25 U/mL], and 26 [50 U/mL] patients in the APO 2 groups; 31 patients in the placebo group) were treated with the respective preparation for 4 weeks.

### Results

The wound area in patients treated with 25 U/ml per cm² wound area could be decreased within 8 weeks of treatment by over 60% in average compared to the lower and even higher doses and the placebo group (see Table 1). Interestingly, in patients having wound areas >5 cm² no significant difference in the decrease of the wound area could be observed in the 12.5/25/50 U/ml groups compared to the placebo group. A minor effect in wound decrease could be observed in patients with wounds having an area of >4 cm² and <5 cm².

**Table 1: Percentage reduction in wound area from baseline (at the beginning of the treatment) to week 4 (patients with adjudicated wound areas ≥0.8 cm² to <4 cm² at baseline, n = 69)**

| | **APO-2 12.5 U/mL/cm²** | **APO-2 25.0 U/mL/cm²** | **APO-2 50.0 U/mL/cm²** | **Placebo** |
|---|---|---|---|---|
| Mean | 53.85 | 60.19 | 46.24 | 18.65 |

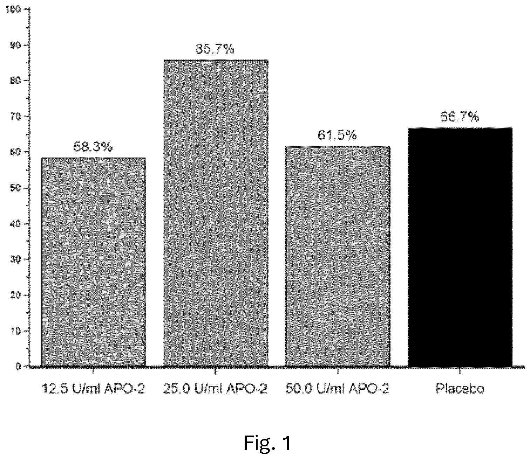

As shown in Fig. 1 85.7 % of the patients treated with 25 U/ml per cm² wound area showed after 4 weeks treatment an at least 80% reduction in wound area.

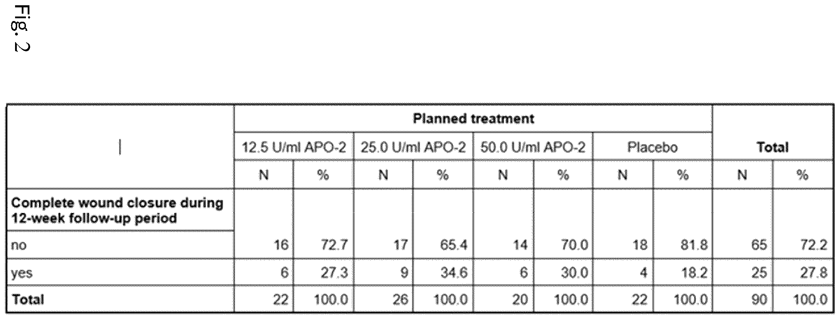

In Fig. 2 the observed complete wound closure in 90 patients who agreed to a follow-up visit after 12 weeks after the last treatment is shown. It turned out that the application of 25 U/ml per cm² wound area showed the best results.

## Claims

1. Pharmaceutical composition comprising a supernatant of a peripheral blood mononuclear cell (PBMC) culture for use in the treatment of a wound and/or for promoting wound healing or wound closure, wherein the supernatant is obtainable by cultivating PBMCs in a culture medium for at least 4 h, wherein the PBMC cell culture is subjected to ionizing radiation at a dose of at least 10 Gy before or during cultivation, wherein the composition is applied topically on the wound at a dose equivalent to a supernatant of 20×10⁶ to 50×10⁶ PBMCs/ml culture medium per cm² wound area.

2. Pharmaceutical composition for the use according to claim 1, wherein the wound is a chronic wound or an acute wound.

3. Pharmaceutical composition for the use according to claim 1 or 2, wherein the wound is a skin wound, preferably an ulcer, a burn, an abrasion, a laceration, a puncture wound, an incision or an avulsion.

4. Pharmaceutical composition for the use according to claim 2 or 3, wherein the chronic wound is an ulcer, preferably a diabetic ulcer, more preferably a diabetic foot ulcer.

5. Pharmaceutical composition for the use according to any one of claims 1 to 4, wherein the wound covers an area of less than 10 cm², preferably of less than 8 cm², more preferably of less than 5 cm², more preferably of less than 4 cm².

6. Pharmaceutical composition for the use according to any one of claims 1 to 5, wherein the wound covers an area of more than 0.1 cm², preferably of more than 0.2 cm², more preferably of more than 0.5 cm², more preferably of more than 0.8 cm², more preferably of more than 1 cm².

7. Pharmaceutical composition for the use according to any one of claims 1 to 6, wherein the composition is administered to the wound for at least 8 days, preferably for at least 10 days, more preferably for at least 14 days, more preferably for at least 21 days, more preferably for at least 30 days.

8. Pharmaceutical composition for the use according to any one of claims 1 to 7, wherein the composition is applied to the wound twice a day, preferably once a day, more preferably every second day, more preferably every third day, more preferably every fourth day, more preferably every fifth day, more preferably every sixth day, more preferably every seventh day.

9. Pharmaceutical composition for the use according to any one of claims 1 to 3, wherein the composition is applied to the wound once a week, preferably twice a week, more preferably three times a week, more preferably four times a week, more preferably five times a week, more preferably six times a week, more preferably daily.

10. Pharmaceutical composition for the use according to any one of claims 1 to 9, wherein the composition is applied topically on the wound at a dose equivalent to a supernatant of 20×10⁶ to 40×10⁶ PBMCs/ml, preferably 20×10⁶ to 30×10⁶ PBMCs/ml, more preferably 22×10⁶ to 28×10⁶ PBMCs/ml, more preferably 24×10⁶ to 26×10⁶ PBMCs/ml, more preferably about 25×10⁶ PBMCs/ml, culture medium per cm² wound area.

11. Pharmaceutical composition for the use according to any one of claims 1 to 10, wherein the composition comprises 0.5 to 5 wt%, preferably 1 to 4 wt% of the alginate, more preferably 2 to 3 wt% of the alginate, more preferably about 2.25 wt%, of an alginate.

12. Pharmaceutical composition for the use according to claim 11, wherein the alginate is a sodium alginate and/or a potassium alginate.

13. Pharmaceutical composition for the use according to any one of claims 1 to 12, wherein the pharmaceutical composition comprises further 0.1 to 3 wt%, preferably 0.5 to 2 wt%, more preferably about 0.825 wt%, cellulose or at least one cellulose derivative.

14. Pharmaceutical composition for the use according to claim 13, wherein the at least on cellulose derivative is carboxymethyl cellulose (CMC) and/or hydroxyethyl cellulose.

15. Pharmaceutical composition for the use according to any one of claims 1 to 14, wherein the pharmaceutical composition comprises further 10 to 25 wt%, preferably 15 to 20 wt%, more preferably about 18.25 wt%, of at least one diol, preferably propane-1,2-diol.
